(19) Europäisches Patentamt
European Patent Office
Office européen des brevets

(11) Veröffentlichungsnummer: **0 005 710**
**A2**

(12) # EUROPÄISCHE PATENTANMELDUNG

(21) Anmeldenummer: 79100963.2

(22) Anmeldetag: 30.03.79

(51) Int. Cl.²: C 07 C 41/04
C 07 C 43/18, C 07 C 43/20
A 01 N 9/24

(30) Priorität: 31.05.78 CH 5957/78

(43) Veröffentlichungstag der Anmeldung: 12.12.79 Patentblatt 79/25

(84) Benannte Vertragsstaaten: BE CH DE FR GB IT LU NL SE

(71) Anmelder: F.Hoffmann-La Roche & Co. Aktiengesellschaft
Abt. VIII-Pt
CH-4002 Basel(CH)

(72) Erfinder: Dorn, Silvia, Dr.
Bohnackerstrasse 5
CH-8157 Dielsdorf(CH)

(72) Erfinder: Pfiffner, Albert, Dr.
Grampenweg 10
CH-8180 Bülach(CH)

(72) Erfinder: Zurflüh, René, Dr.
Dachslenbergstrasse 54
CH-8180 Bülach(CH)

(74) Vertreter: Hofmann, Dieter, Dr. et al,
Grenzacherstrasse 124 Postfach 601
CH-4002 Basel(CH)

(54) **Propinyloxymethyl-derivat, Herstellung dieser Verbindung sowie Schädlingsbekämpfungsmittel, die diese Verbindung beziehungsweise ein Analoges enthalten und die Verwendung beider Verbindungen als Schädlingsbekämpfungsmittel.**

(57) Die Erfindung betrifft Schädlingsbekämpfungsmittel die als Wirkstoffe eine Verbindung der Formel

$HC{\equiv}C\text{-}H_2C\text{-}O\text{-}H_2C\text{-}X\text{-}CH_2\text{-}O\text{-}CH_2\text{-}C{\equiv}CH$ I,a

worin X einen Phenylen- oder einen Cyclo-hexylenrest bedeutet,

sowie inertes Trägermaterial enthalten. Ferner betrifft die Erfindung die neue Verbindung der Formel

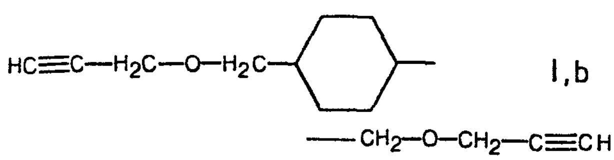

I,b

die dadurch hergestellt wird, dass man

a) eine Verbindung der Formel

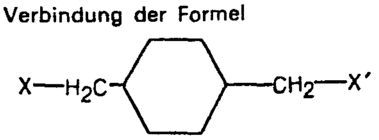

II

worin X und X' Chlor, Brom, Jod, Mesyloxy oder Tosyloxy bedeuten,

mit mindestens 2 Molen einer Verbindung der Formel

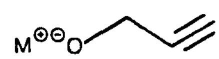

III

worin $M^{\oplus}$ ein Alkali- oder Erdalkali-Ion bedeutet, umsetzt, oder

b) eine Verbindung der Formel

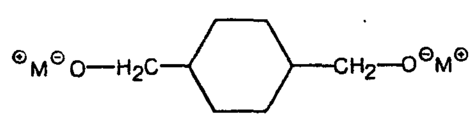

IV

worin $M^{\oplus}$ die in der Formel III angegebene Bedeutung besitzt,

mit einer Verbindung der Formel

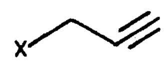

V

worin X die in Formell II angegebene Bedeutung besitzt, umsetzt.

EP 0 005 710 A2

Croydon Printing Company Ltd.

0005710

F. Hoffmann-La Roche & Co. Aktiengesellschaft, Basel, Schweiz

RAN 6101/87

Propinyloxymethyl-derivat, Herstellung dieser Verbindung sowie Schädlingsbekämpfungsmittel, die diese Verbindung beziehungsweise ein Analoges enthalten und die Verwendung beider Verbindungen als Schädlingsbekämpfungsmittel.

Die Erfindung betrifft Schädlingsbekämpfungsmittel die als Wirkstoffe eine Verbindung der Formel

$$HC\equiv C-H_2C-O-H_2C-X-CH_2-O-CH_2-C\equiv CH \qquad I,a$$

worin X einen Phenylen- oder einen Cyclohexylenrest bedeutet,

sowie inertes Trägermaterial enthalten. Ferner betrifft die Erfindung die neue Verbindung der Formel

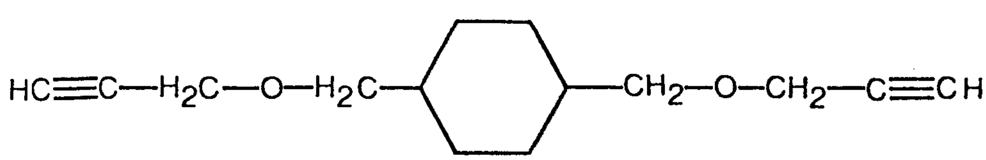

I,b

die dadurch hergestellt wird, dass man

a) eine Verbindung der Formel

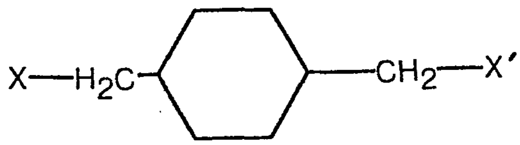

II

worin X und X' Chlor, Brom, Jod, Mesyloxy oder Tosyloxy bedeuten,

mit mindestens 2 Molen einer Verbindung der Formel

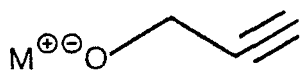

III

worin $M^{\oplus}$ ein Alkali- oder Erdalkali-Ion bedeutet,

umsetzt, oder

b) eine Verbindung der Formel

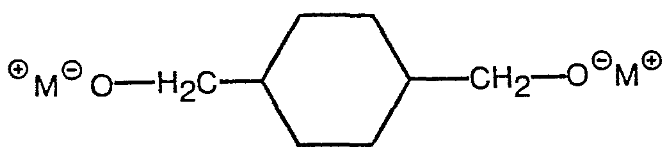

IV

worin $M^{\oplus}$ die in der Formel III angegebene Bedeutung besitzt,

mit einer Verbindung der Formel

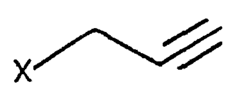

V

worin X die in Formel II angegebene Bedeutung besitzt,

umsetzt.

Gemäss Verfahrensvariante a) erfolgt die Umsetzung eines Alkoholates der Formel III mit einer Verbindung der Formel II in einem inerten organischen Lösungsmittel, vorzugsweise in Dimethylformamid, Dioxan, Hexamethylphosphorsäuretriamid, Tetrahydrofuran, Dimethoxyäthan oder in einer Kombination zweier oder mehrerer dieser Lösungsmittel.

Zweckmässigerweise wird der entsprechende Alkohol eingesetzt und zwar in Gegenwart eines Alkalimetalls oder Erdalkalimetalls, eines entsprechenden Hydrides oder Amides oder eines Alkalimetallhydroxydes. Hierbei bildet sich aus dem Alkohol das entsprechende Alkoholat. Bevorzugte Alkali- oder Erdalkalimetalle sind Natrium und Kalium bzw. Calcium und Magnesium. Der Reaktionstemperatur kommt keine entscheidende Bedeutung zu. Sie kann zweckmässigerweise zwischen $0^{o}$-$20^{o}C$ und der Siedetemperatur des Reaktionsgemisches liegen. Eine bevorzugte Reaktionstemperatur ist 50-$70^{o}C$, insbesondere für den Fall, dass X und X' in Formel II in der Bedeutung Brom vorliegen.

Die Umsetzung einer Verbindung der Formel IV mit einer Verbindung der Formel V gemäss Verfahrensvariante b) erfolgt unter den gleichen Bedingungen wie vorstehend für die Verfahrensvariante a) beschrieben.

Die bevorzugte Verbindung der Formel I ist alpha, alpha'-Bis(2-propinyloxy)-p-xylol.

Die Verbindungen der Formel I sind Wirkstoffe für Schädlingsbekämpfungsmittel und eignen sich insbesondere zur Bekämpfung von Insekten. Somit umfasst die Erfindung auch Verfahren zur Herstellung derartiger Mittel sowie deren Verwendung.

Die pestizide Zusammensetzung gemäss vorliegender Erfindung enthält zweckmässigerweise zumindest eines der folgenden Materialien: Trägerstoffe, Netzmittel, inerte Verdünnungsmittel und Lösungsmittel.

Die vorliegende Erfindung betrifft ferner die Behandlung von Pflanzen, Tieren und des Erdbodens sowie von Gegenständen und Flächen, welche darin besteht, dass auf diese eine Verbindung der Formel I, wie oben definiert, aufgebracht wird.

Die Verbindungen der Formel I sind somit ganz allgemein als Pestizide wertvoll. Sie zeigen sich besonders wertvoll als Insektizide und Akarizide, unter anderem gegen Coleoptera wie z.B. Epilachna spp. und Leptinotarsa decemlineata; gegen Homoptera, insbesondere gegen Vertreter aus der Familie Aleyrodidae wie z.B. Trialeurodes vaporariorum, Aleyrodes proletella, Aleyrothrixus floccosus, Dialeurodes citrifolii, Bemisia tabaci und Aleurocanthus woglumi, ferner gegen Vertreter weiterer Homopteren-Familien wie Planococcus spp. und Psylla spp; gegen Lepidoptera wie z.B. Spodoptera littoralis, Adoxophyes reticulana, Ostrinia nubilalis, Heliothis virescens und Miniermotten; ferner gegen Acarina wie z.B. Tetranychus urticae und Panonychus ulmi und tetrapodile Milben. Die Verbindungen wirken als direkte Insektizide und Akarizide und haben auch eine gewisse systemische Aktivität. Die Wirkung dieser Verbindungen über die Dampfphase stellt einen bevorzugten Aspekt der vorliegenden Erfindung dar. Dies gilt insbesondere für die ovizide Wirkung der Verbindungen der Formel I.

Die Verbindungen der Formel I sind im allgemeinen wasserunlöslich und können nach irgendeiner der für unlösliche Verbindungen üblichen Methoden konfektioniert werden.

Wenn gewünscht, können die Verbindungen der Formel I in einem mit Wasser nicht mischbaren Lösungsmittel, wie beispielsweise einem hochsiedenden Kohlenwasserstoff, gelöst werden, das zweckmässigerweise gelöste Emulgatoren enthält, so dass es bei Zugabe von Wasser als selbstemulgierbares Oel wirkt.

Die Verbindungen der Formel I können auch mit einem Netzmittel mit oder ohne inertes Verdünnungsmittel zur Bildung eines netzbaren Pulvers vermischt werden, welches in Wasser löslich oder dispergierbar ist, oder sie können mit dem inerten Verdünnungsmittel zu Bildung eines festen oder pulverförmigen Produktes vermischt werden.

Inerte Verdünnungsmittel, mit welchen die Verbindungen der Formel I verarbeitet werden können, sind feste inerte Medien, einschliesslich pulverförmige oder feinverteilte Feststoffe, wie beispielsweise Tone, Sande, Talk, Glimmer, Düngemittel und dgl., wobei solche Produkte entweder staubförmig oder als Materialien mit grösserer Teilchengrösse vorliegen können.

Die Netzmittel können anionische Verbindungen, wie beispielsweise Seifen, Fettsulfatester, wie Dodecylnatriumsulfat, Octadecylnatriumsulfat und Cetylnatriumsulfat, fettaromatische Sulfonate, wie Alkylbenzolsulfonate oder Butylnaphthalinsulfonate, komplexere Fettsulfonate, wie die Amidkondensationsprodukte von Oelsäure und N-Methyltaurin oder das Natriumsulfonat von Dioctylsuccinat sein.

Die Netzmittel können auch nichtionische Netzmittel, wie beispielsweise Kondensationsprodukte von Fettsäuren, Fettalkoholen oder fettsubstituierten Phenolen mit Aethylenoxyd, oder Fettsäureester und -äther von Zuckern oder mehrwertigen Alkoholen oder die Produkte, die aus letzteren durch Kondensation mit Aethylenoxyd erhalten werden, oder die Produkte, die als Blockcopolymere von Aethylenoxyd und Propylenoxyd bekannt sind, sein. Die Netzmittel können auch kationische Mittel, wie beispielsweise Cetyltrimethylammoniumbromid und dgl., sein.

Die pestizide Zusammensetzung kann auch in Form einer Aerosolverbindung vorliegen, wobei zweckmässigerweise zusätzlich zum Treibgas, welches geeigneterweise ein polyhalogeniertes Alkan, wie Dichlordifluormethan ist, ein Co-Solvens und ein Netzmittel verwendet wird.

Die pestiziden Zusammensetzungen, gemäss der vorliegenden Erfindung können zusätzlich zu den Verbindungen der Formel I, Synergisten und andere aktive Insektizide, Bakterizide und Fungizide enthalten.

In ihren verschiedenen Anwendungsgebieten können die erfindungsgemässen Verbindungen in unterschiedlichen Mengenverhältnissen eingesetzt werden; so werden beispielsweise für die Behandlung von Pflanzen zur Bekämpfung von Schädlingen auf diesen die Verbindungen zweckmässigerweise in einem Ausmass von etwa 100-2000 g/ha verwendet; für die Behandlung von Tieren zur Bekämpfung von Ectoparasiten wird das Tier zweckmässigerweise in eine Lösung, enthaltend 10-500 ppm aktiver Verbindung, getaucht oder mit dieser besprüht.
Die Toxizität der Verbindungen der Formel I liegt bei über 1000 mg/kg (akute Toxizität an der Maus).

Die Verbindungen der Formel I,a können sowohl als cis/trans-Gemisch als auch in Form der cis-Verbindung und/oder der trans-Verbindung vorliegen.

Die folgenden Beispiele sollen die vorliegende Erfindung illustrieren.

## Beispiel 1

303,75 g 55%ige Natriumhydrid-Suspension werden dreimal mit 200 ml Hexan gewaschen, mit 990 ml abs. Dimethylformamid überschichtet und unter Stickstoffbegasung bei Raumtemperatur während einer Stunde mit einer Lösung von 502 g cis/trans-1,4-Bis(hydroxymethyl)-cyclohexan in 2000 ml abs. Dimethylformamid versetzt und bis zur Beendigung der Wasserstoffentwicklung bei 40°C gerührt. Bei Raumtemperatur werden anschliessend 910 g Propargylbromid langsam zugetropft, wobei die Reaktionstemperatur auf ca. 38°C steigt. Nach 16-stündigem Nachrühren bei 40°C wird auf Raumtemperatur abgekühlt und das Reaktionsgemisch unter guter Stickstoffspülung vorsichtig mit 350 ml Essigsäure versetzt und auf Eiswasser gegossen. Das Reaktionsprodukt wird mit Methylenchlorid erschöpfend extrahiert, mit Wasser neutralgewaschen, getrocknet und eingedampft. Durch fraktionierte Destillation wird reines 1,4-Bis-[(2-propinyloxy)-methyl]-cyclohexan (cis,trans-Gemisch) erhalten; Sdp. 116°C/0,05 Torr; Smp. 56-60°C.

## Beispiel 2

Alpha,alpha'-Bis(2-propinyloxy)-p-xylol kann wie folgt formuliert werden:

| | | g/l |
|---|---|---|
| Wirkstoff | | 500,0 |
| Mischung von Rizinusöl/Aethylenoxid-Kondensationsprodukt mit ca. 25 Mol Aethylenoxyd und Ca-Dodecylbenzolsulfonat im Verhältnis 3:1 | | 100,0 |
| Epoxidiertes Soyaöl mit einem Oxiran-Sauerstoffgehalt von 6% | | 25,0 |
| Butyliertes Hydroxytoluol | | 10,0 |
| Lösungsmittel bestehend aus einer Mischung aus Mono-, Di- und Trinieder Alkylbenzolen | ad | 1000 ml |

## Beispiel 3

| | |
|---|---|
| 1,4-Bis-[(2-propinyloxy)-methyl]-cyclohexan | 50,0 g |
| Hochdisperse Kieselsäure | 5,0 g |
| Natrium-laurylsulfat | 1,0 g |
| Natrium-lignosulfonat | 2,0 g |
| Kaolin | 42,0 g |
| | 100,0 g |

## Beispiel 4

| | g/l |
|---|---|
| 1,4-Bis-[(2-propinyloxy)-methyl]-cyclohexan | 250 |
| N-Methyl-2-pyrrolidon | 300 |
| Mischung von Rizinusöl/Aethylenoxid-Kondensationsprodukt mit ca. 25 Mol Aethylenoxyd und Calcium-Dodecylbenzolsulfonat im Verhältnis 3:1 | 50 |
| Cycloalkylepoxistearat | 25 |
| Lösungsmittel bestehend aus einer Mischung aus Mono-, Di- und Tri-nieder Alkylbenzolen | ad 1000 ml |

## Beispiel 5

| | |
|---|---|
| 1,4-Bis-[(2-propinyloxy)-methyl]-cyclohexan | 25,0 g |
| Hochdisperse Kieselsäure | 5,0 g |
| Fossile Kieselsäure | 25,0 g |
| Natrium-laurylsulfat | 1,0 g |
| Natrium-lignosulfonat | 2,0 g |
| Kaolin | 42,0 g |
| | 100,0 g |

Strukturformel und Nomenklatur der in den nachstehenden Beispielen erwähnten Verbindungen:

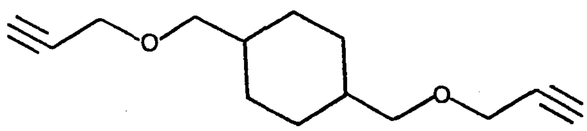

1,4-Bis-[(2-propinyloxy)-methyl]-cyclohexan (cis/trans-Mischung)

Wirkstoff A

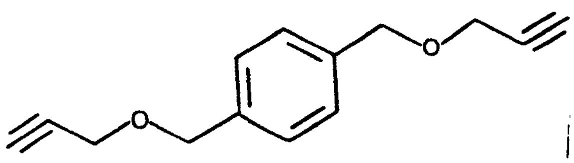

alpha,alpha'-Bis(2-propinyloxy)-p-xylol

Wirkstoff B

Chlordimeform

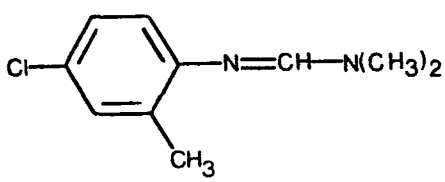

N'-(4-Chlor-2-methylphenyl)-N,N-dimethylformamidin

Zinophos

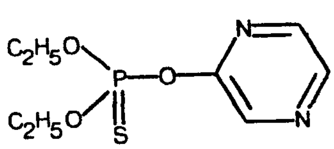

O,O-Diäthyl-O-(pyrazin-2-yl)-monothiophosphat

## Beispiel 6

Testinsekt: Trialeurodes vaporariorum, Weisse Fliege

Testmethode: Ovizidversuch (Laborversuch)

Mit Eiern infizierte Buschbohnen werden mit einer wässrigen Spritzbrühe des Wirkstoffes behandelt. 7 Tage nach der Behandlung wird die Schlupfrate bestimmt. Die Resultate werden ausgedrückt als prozentuale Reduktion der Schlupfrate im Vergleich zur unbehandelten Kontrolle.

| Formulierung gemäss Beispiel | Wirkstoff | Konz. % ai | % Wirkung |
|---|---|---|---|
| 4 | A | 0,1 | 100 |
| | | 0,03 | 100 |
| | | 0,01 | 100 |
| | | 0,003 | 44 |
| | | 0,001 | 7 |
| 3 | A | 0,1 | 100 |
| | | 0,03 | 100 |
| | | 0,01 | 100 |
| | | 0,003 | 89 |
| | | 0,001 | 20 |
| 2 | B | 0,1 | 100 |
| | | 0,03 | 100 |
| | | 0,01 | 100 |
| | | 0,003 | 75 |
| | | 0,001 | 45 |
| | Zinophos | 0,1 | 0 |
| | NEMAPHOS | 0,03 | 0 |
| | | 0,01 | 0 |
| | | 0,003 | 0 |
| | | 0,001 | 0 |

(Unbehandelte Kontrollparzellen: 8% natürliche Mortalität der Eier)

## Beispiel 7

Testinsekt: Aleurodes proletella, Weisse Fliege

Testmethode: Ovolarvizidversuch (Freilandversuch)

Rosenkohl mit einer grossen Population von Aleurodes proletella wird mit einer wässrigen Spritzbrühe des Wirkstoffes gespritzt. Nach 4 Wochen wird nach jungen Larven ausgezählt. Die Resultate werden ausgedrückt als prozentuale Reduktion der Population (Ovolarvizide Wirkung) im Vergleich zur unbehandelten Kontrolle.

| Formulierung gem. Beispiel | Wirkstoff | Konz. % ai | % Wirkung |
|---|---|---|---|
| 5 | A | 0,1 | 98 |
| | | 0,05 | 90 |
| | | 0,025 | 97 |
| 2 | B | 0,1 | 100 |
| | | 0,05 | 96 |
| | | 0,025 | 97 |
| | Zinophos | 0,1 | 0 |
| | NEMAPHOS | 0,05 | 0 |
| | | 0,025 | 0 |

(Population bei unbehandelter Kontrolle: je 486 Larven)

## Beispiel 8

Testinsekt: Epilachna chrysomelina, Gurkenkäfer

Testmethode: Ovizidversuch (Laborversuch)

Kürbispflanzen mit frisch gelegten Epilachna-Eiern werden mit einer wässrigen Spritzbrühe des Wirkstoffes gespritzt. Nach 6 Tagen wird die Schlupfrate bestimmt. Die Resultate werden ausgedrückt als prozentuale Reduktion der Schlupfrate im Vergleich zur unbehandelten Kontrolle.

| Formulierung gem. Beispiel | Wirkstoff | Konz. % ai | % Wirkung |
|---|---|---|---|
| 3 | A | 0,1 | 100 |
| | | 0,03 | 100 |
| | | 0,01 | 93 |
| | | 0,003 | 61 |
| | | 0,001 | 13 |
| 4 | A | 0,1 | 100 |
| | | 0,03 | 57 |
| | | 0,01 | 3 |
| | | 0,003 | 0 |
| | | 0,001 | 0 |

| | | | |
|---|---|---|---|
| 2 | B | 0,1 | 100 |
| | | 0,03 | 94 |
| | | 0,01 | 55 |
| | | 0,003 | 3 |
| | | 0,001 | 4 |
| | Chlordimeform GALECRON | 0,1 | 0 |
| | | 0,03 | 0 |
| | | 0,01 | 0 |
| | | 0,003 | 0 |
| | | 0,001 | 0 |

(Unbehandelte Kontrolle: 8% natürliche Mortalität der Eier)

## Beispiel 9

Testinsekt: Epilachna chrysomelina, Gurkenkäfer

Testmethode: Ovizid-Gasphase-Versuch (Laborversuch)

Deckel von Petrischalen werden mit einer wässrigen Spritzbrühe des Wirkstoffes behandelt. Nach dem Abtrocknen des Spritzbelages wird ein Eigelege auf den Boden der Petrischale gelegt. Mit dem behandelten Deckel wird die Schale verschlossen. Nach 6 Tagen wird die Schlupfrate bestimmt. Die Resultate werden ausgedrückt als prozentuale Reduktion der Schlupfrate im Vergleich zur unbehandelten Kontrolle.

| Formulierung gem. Beispiel | Wirkstoff | Konz. % ai | % Wirkung |
|---|---|---|---|
| 4 | A | 0,1 | 100 |
| | | 0,03 | 44 |
| | | 0,01 | 0 |
| | | 0,003 | 0 |
| | | 0,001 | 0 |
| 2 | B | 0,1 | 100 |
| | | 0,03 | 100 |
| | | 0,01 | 88 |
| | | 0,003 | 65 |
| | | 0,001 | 13 |
| | Chlordimeform GALECRON | 0,1 | 0 |
| | | 0,03 | 0 |
| | | 0,01 | 0 |
| | | 0,003 | 0 |
| | | 0,001 | 0 |

(Unbehandelte Kontrolle: 7% natürliche Mortalität der Eier)

## Beispiel 10

Testinsekt: Ostrinia nubilalis, Maiszünsler

Testmethode: Ovizidversuch (Laborversuch)

Maispflanzen mit frisch gelegten Ostrinia-Eiern werden mit einer wässrigen Spritzbrühe des Wirkstoffes gespritzt. Nach 6 Tagen wird die Schlupfrate bestimmt. Die Resultate werden ausgedrückt als prozentuale Reduktion der Schlupfrate im Vergleich zur unbehandelten Kontrolle.

| Formulierung gem. Beispiel | Wirkstoff | Konz. % ai | % Wirkung |
|---|---|---|---|
| 4 | A | 0,1 | 52 |
| | | 0,01 | 0 |
| 2 | B | 0,1 | 100 |
| | | 0,01 | 37 |
| | Chlordimeform | 0,1 | 28 |
| | GALECRON | 0,01 | 7 |

(Unbehandelte Kontrolle: 7% natürliche Mortalität der Eier)

## Beispiel 11

Testinsekt: Leptinotarsa decemlineata, Kartoffelkäfer

Testmethode: Ovizidversuch (Laborversuch)

Kartoffelpflanzen mit frisch gelegten Leptinotarsa-Eiern werden mit einer wässrigen Spritzbrühe des Wirksstoffes gespritzt. Nach 6 Tagen wird die Schlupfrate bestimmt. Die Resultate werden ausgedrückt als prozentuale Reduktion der Schlupfrate im Vergleich zur unbehandelten Kontrolle.

| Formulierung gem. Beispiel | Wirkstoff | Konz. % ai | % Wirkung |
|---|---|---|---|
| 3 | A | 0,1 | 86 |
| | | 0,03 | 0 |
| | | 0,01 | 0 |
| 2 | B | 0,1 | 100 |
| | | 0,03 | 41 |
| | | 0,01 | 0 |
| | Chlordimeform GALECRON | 0,1 | 87 |

(Unbehandelte Kontrolle: 1% natürliche Mortalität der Eier)

## Beispiel 12

Testinsekt: Leptinotarsa decemlineata, Kartoffelkäfer

Testmethode: Ovizid-Gasphase-Versuch (Laborversuch)

Deckel von Petrischalen werden mit einer wässrigen Spritzbrühe des Wirkstoffes behandelt. Nach dem Abtrocknen des Spritzbelages wird ein Eigelege auf den Boden der Petrischale gelegt. Mit dem behandelten Deckel wird die Schale verschlossen. Nach 6 Tagen wird die Schlupfrate bestimmt. Die Resultate werden ausgedrückt als prozentuale Reduktion der Schlupfrate im Vergleich zur unbehandelten Kontrolle.

| Formulierung gem. Beispiel | Wirkstoff | Konz. % ai | % Wirkung |
|---|---|---|---|
| 4 | A | 0,1 | 100 |
| | | 0,03 | 0 |
| | | 0,01 | 0 |
| | | 0,003 | 0 |
| | | 0,001 | 0 |
| 2 | B | 0,1 | 100 |
| | | 0,03 | 100 |
| | | 0,01 | 79 |
| | | 0,003 | 19 |
| | | 0,001 | 49 |
| | Chlordimeform GALECRON | 0,1 | 83 |
| | | 0,03 | 19 |
| | | 0,01 | 1 |
| | | 0,003 | 0 |
| | | 0,001 | 0 |

(Unbehandelte Kontrolle: 1% natürliche Mortalität der Eier)

## Beispiel 13

Testinsekt: Spodoptera littoralis, Baumwolleule

Testmethode: Ovizid-Gasphase-Versuch (Laborversuch)

Petrischalendeckel werden mit einer wässrigen Spritzbrühe des Wirkstoffes behandelt und auf den Boden eines Exsikkators gelegt. Dann werden die Exsikkatoren verschlossen. In verschiedenen Zeitintervallen nach der Behandlung werden Eigelege von Spodoptera littoralis in den Exsikkator gehängt und für 6 Stunden der Wirkstoffatmosphäre ausgesetzt. Nach 6 Tagen wird die Schlupfrate bestimmt. Die Resultate werden ausgedrückt als prozentuale Reduktion der Schlupfrate im Vergleich zur unbehandelten Kontrolle.

| Formulierung gem. Beispiel | Wirkstoff | mg ai/$m^3$ | % Wirkung nach x Tagen 0 | 14 | 22 | 35 | 49 | 56 Tage |
|---|---|---|---|---|---|---|---|---|
| 2 | B | 150 | 100 | 100 | 100 | 100 | 100 | 53 |
| | Chlordimeform GALECRON | 150 | 100 | 59 | 30 | 0 | 0 | 0 |
| (Unbehandelte Kontrolle: | | | 0 | 2 | 3 | 0 | 0 | 2 |
| | | | % natürliche Mortalität der Eier) | | | | | |

Chlordimeform (GALECRON), EC (500), Handelsformulierung bezogen bei Ciba-Geigy.

Zinophos (NEMAPHOS), EC (500), Handelsformulierung bezogen bei Dr. R. Maag AG.

Patentansprüche

1. 1,4-Bis-[(2-propinyloxy)-methyl]-cyclohexan.

2. 1,4-Bis-[(2-propinyloxy)-methyl]-cyclohexan als Schädlingsbekämpfungsmittel.

3. Schädlingsbekämpfungsmittel, dadurch gekennzeichnet, dass es mindestens eine Verbindung der allgemeinen Formel

$$HC{\equiv}C\text{-}H_2C\text{-}O\text{-}H_2C\text{-}X\text{-}CH_2\text{-}O\text{-}CH_2\text{-}C{\equiv}CH \qquad 1,a$$

worin X einen Phenylen- oder einen Cyclohexylenrest bedeutet,

sowie inertes Trägermaterial enthält.

4. Schädlingsbekämpfungsmittel gemäss Anspruch 3, dadurch gekennzeichnet, dass es alpha, alpha'-Bis(2-propinyloxy)-p-xylol enthält.

5. Verfahren zur Herstellung von Verbindungen der allgemeinen Formel

$$HC{\equiv}C{-}H_2C{-}O{-}H_2C{-}X{-}CH_2{-}O{-}CH_2{-}C{\equiv}CH \qquad 1,a$$

worin X einen Phenylen- oder einen Cyclohexylenrest bedeutet,

dadurch gekennzeichnet, dass man

a) eine Verbindung der Formel

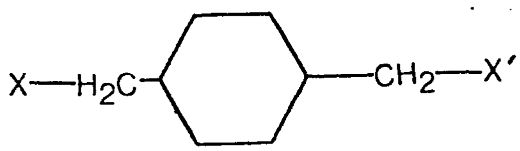

II

worin X und X' Chlor, Brom, Jod, Mesyloxy oder Tosyloxy bedeuten,

mit mindestens 2 Molen einer Verbindung der Formel

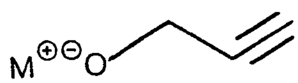

III

worin $M^{\oplus}$ ein Alkali- oder Erdalkali-Ion bedeutet,

umsetzt, oder

b) eine Verbindung der Formel

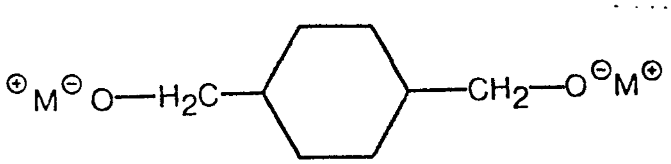

IV

worin $M^{\oplus}$ die in der Formel III angegebene Bedeutung besitzt,

mit einer Verbindung der Formel

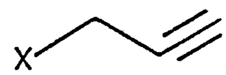

V

worin X die in Formel II angegebene Bedeutung besitzt,

umsetzt.

6. Verfahren zur Bekämpfung von Schädlingen, dadurch gekennzeichnet, dass man die zu schützenden Gegenstände mit einem in den Ansprüchen 3 und 4 genannten Mittel behandelt.

7. Verwendung der in den Ansprüchen 3 und 4 genannten Verbindungen als Schädlingsbekämpfungsmittel.

***